**Europäisches Patentamt**

**European Patent Office**

**Office européen des brevets**

(19)

(11) Veröffentlichungsnummer : **0 073 513 B2**

(12) # NEUE EUROPÄISCHE PATENTSCHRIFT

(45) Veröffentlichungstag der neuen Patentschrift :
**05.02.92 Patentblatt 92/06**

(51) Int. Cl.⁵ : **G01N 33/52,** G01N 31/22, G01N 21/07

(21) Anmeldenummer : **82107972.0**

(22) Anmeldetag : **30.08.82**

(54) **Verfahren zur Durchführung analytischer Bestimmungen und hierfür geeignetes Mittel.**

(30) Priorität : **01.09.81 DE 3134611**

(43) Veröffentlichungstag der Anmeldung :
**09.03.83 Patentblatt 83/10**

(45) Bekanntmachung des Hinweises auf die
Patenterteilung :
**24.07.85 Patentblatt 85/30**

(45) Bekanntmachung des Hinweises auf die
Entscheidung über den Einspruch :
**05.02.92 Patentblatt 92/06**

(84) Benannte Vertragsstaaten :
**AT BE CH DE FR GB IT LI LU NL SE**

(56) Entgegenhaltungen :
**EP-A- 0 014 797**
**DE-A- 1 944 246**
**DE-A- 2 536 886**
**DE-A- 2 927 345**
**US-A- 3 999 868**
**US-A- 4 244 916**

(73) Patentinhaber : **BOEHRINGER MANNHEIM GMBH**
**Sandhofer Strasse 116**
**W-6800 Mannheim 31 (DE)**

(72) Erfinder : **Klose, Sigmar, Dr.**
**Breitenloh 7**
**W-8131 Berg 2 (DE)**
Erfinder : **Stähler, Fritz, Dr.**
**Heimgartenstrasse 4**
**W-8132 Tutzing (DE)**

(74) Vertreter : **Weickmann, Heinrich, Dipl.-Ing. et al**
**Patentanwälte H.Weickmann, Dr. K.Fincke F.A. Weickmann, B. Huber Dr. H. Liska, Dr. J. Prechtel Kopernikusstrasse 9 Postfach 86 08 20**
**W-8000 München 86 (DE)**

EP 0 073 513 B2

## Beschreibung

Die Erfindung betrifft ein Verfahren zur Durchführung analytischer Bestimmungen durch Mischen und Inkubieren einer Probelösung mit wenigstens einem Reagenz und Messung eines Parameters im Reaktionsgemisch und ein hierfür geeignetes Mittel.

Die Verwendung von Trockenreagentien auf geeigneten inerten Trägermaterialien ist ein seit langem bekanntes Hilfsmittel bei der Durchführung chem. Reaktionen, die zum qualitativen oder quantitativen Nachweis einer zu analysierenden Substanz herangezogen werden können. Als Beispiele seien genannt: DE-AS 2 332 760, DE-OS 2 717 817, EPA 0 014 797, DE-OS 2 752 352, DE-OS 2 927 345. Diesen Verfahren ist gemeinsam, dass die in Lösung befindliche Probe auf den Reagenzträger gegeben wird. Von seinem Auftragsort diffundiert die Probe dann unter Einwirkung von Kapillarkräften in den Träger.

Auf dem Weg werden Reagentien ganz oder teilweise aufgelöst, die auf diese Weise gebildete Reagenz-Probelösung wandert weiter, bis sie schliesslich zu einer Messzone gelangt, wo die Farbintensitätsänderung optisch vermessen wird. Die Messzone ist jeweils integraler Bestandteil des Reagenzträgers.

Wie nun z.B. aus der DE-OS 2 927 345 zu entnehmen ist, werfen Verfahren, bei denen die Diffusionsprozesse ungesteuert ablaufen und bei denen die Remission von Lichtstrahlen direkt auf dem Reagenzträger, der im allgemeinen aus einer opaken Schicht besteht, gemessen wird, beträchtliche Probleme auf. Fasermaterialien zeigen Unregelmässigkeiten, die im Mikrobereich zu unterschiedlichen Ausbreitungsgeschwindigkeiten der Flüssigkeiten führen, es entstehen Zonen mit höheren oder niedrigeren Reagenzkonzentrationen als sie optimal sind. So wird z.B. in der EPA 0 014 797 erwähnt, dass « Lufttaschen » in den Trägermaterialien zu zusätzlichen Schwierigkeiten führen und die Schlussfolgerung gezogen, dass Flüssigkeitsströme, die durch Kapillarkräfte erzeugt werden, « kontrolliert » werden müssen.

Der zweite wesentliche Nachteil liegt in der Remissionsmessung selbst:
im Gegensatz zur Durchlicht-Fotometrie gibt es hier keine lineare Beziehung zwischen der Konzentration einer lichtabsorbierenden Substanz und der Extinktion. Man erhält mehr oder weniger stark gekrümmte Eichkurven, in die die Oberflächeneigenschaften stark mit eingehen. Darin ist ein grundsätzlicher Nachteil zu sehen, der die Reproduzierbarkeit eines analytischen Auswerteverfahrens stark negativ beeinflusst. Ausserdem können damit Tests, die auf der Messung von sich bildenden oder abnehmenden Trübungen (turbidimetrische Verfahren) beruhen, grundsätzlich nicht durchgeführt werden. Diese stellen aber bei immunologischen Methoden und auch bei Enzymbestimmungen wie der Lipase-Bestimmung eine weit verbreitete zuverlässige Technik dar.

Ein weiterer Nachteil solcher Analysenelemente ist darin zu sehen, dass unterschiedliche Reaktionsphasen bei mehrstufiger Reaktion auf getrennten Schichten der Analysenelemente nicht gezielt angesteuert werden können. Das heisst, die Startzeitpunkte von Folgereaktionen hängen von der nicht konstanten Diffusionsgeschwindigkeit der Lösung ab.

Auch in der schichtenförmigen Anordnung selbst ist ein Nachteil zu sehen, da die Berührungsflächen von Reagenzien, die sich in bezug auf ihre Stabilität ungünstig beeinflussen können, relativ gross sind. Günstiger wäre es, solche Reagenzien streng getrennt voneinander in einem Reagenzträger anzuordnen.

Aus obigem geht hervor, dass es im Sinne der Erzielung von Analysenergebnissen mit maximaler Richtigkeit und Reproduzierbarkeit notwendig ist, die genannten Nachteile zu vermeiden, da sie dem Anwendungsbereich solcher « Analysenelemente », die aus Reagenzträgern aufgebaut sind, relativ enge Grenzen setzen.

Aus der Sicht des Benutzers sind solche Testdurchführungstechniken jedoch insofern vorteilhaft, als sie eine sehr einfache Handhabung erlauben, da keine Reagenzlösungen angesetzt werden müssen, da das Pipettieren von Reagenz entfällt, keine Stabilitätsprobleme mit den in Lösung immer instabileren Reagenzien auftreten usw.

Aus DE-OS 1 944 246 ist es bekannt, aus einer Probelösung mit Hilfe eines speziellen Filtermittels ausgefällte Substanzen wie z.B. Eiweiss, unter Anwendung von Zentrifugalkräften abzutrennen. Aus DE-OS 2 536 886 ist ein Verfahren zur Analyse von Bestandteilen einer flüssigen Probe auf einem Fasermedium bekannt, bei dem auf dem Fasermedium an der Analysenstelle ein reproduzierbarer Kompressionszustand aufrechterhalten wird.

In EPA 0 039 825 wird ein Küvettenrotor für ein Analysegerät und dessen Verwendung beschrieben. Dabei wird eine Flüssigkeit zur genauen Dosierung in Abmeßräume gebracht, die dann durch Erhöhung der Zentrifugalkraft in die Meßküvetten transportiert wird. Die Kapillaren dienen dabei nicht einem Transport der Flüssigkeit, sondern nur dem genauen Dosieren.

Der Erfindung liegt nun die Aufgabe zugrunde, diese Vorteile beizubehalten und gleichzeitig die geschilderten Nachteile zu beseitigen.

Gelöst wird diese Aufgabe erfindungsgemäss durch ein Verfahren zur Durchführung analytischer Bestimmungen durch Mischen und Inkubieren einer Probelösung mit wenigstens einem Reagenz und Messung eines Parameters im Reaktionsgemisch, wobei die Probelösung von einer Aufgabestelle zu einer Messstelle transportiert wird, welches dadurch gekennzeichnet ist, dass man die Probelösung zuerst zu einem löslichen Trockenreagenz transportiert unter wenigstens teilweiser Auflösung des letzteren und dann zur Messstelle weitertransportiert und der Transport duch zwei verschiedene Kräfte erfolgt, wobei er wenigstens auf einem Teil der Transportstrecke durch eine auf die Lösung wirkende Grenzflächenkraft als erste Kraft bewirkt wird, der zur Regelung der Transportgeschwindigkeit oder Transportrichtung als zweite Kraft eine Zentrifugalkraft oder mechanische Druckkraft überlagert wird, die je nachdem, welcher Transportzustand der Flüssigkeit eingestellt werden soll, grösser oder keiner als die erste Kraft gemacht wird.

Das neue Verfahren verbindet die Vorteile der geschilderten Reagenzträgertechnik mit der Genauigkeit und Fehlerfreiheit üblicher nasschemischer Verfahren.

Erreicht wird dies dadurch, dass die zu analysierende Probelösung (im allgemeinen mit Wasser verdünnt) in eine Eingabestelle gegeben wird, von der aus sie auf dem Weg zu einer Messstelle einen oder mehrere Träger von Trockenreagenz durchströmt, wobei die Reagenzien ganz oder teilweise gelöst werden. Das Durchströmen geschieht unter strenger Kontrolle der Fliessgeschwindigkeiten und damit der Fliesszeiten, indem der treibenden Grenzflächenkraft eine zweite Kraft überlagert wird, die den Strom beschleunigen, bremsen oder anhalten kann. Am Ende des Strömungsweges gelangt die Flüssigkeit dann an eine Messstelle, die nicht mit dem Reagenzträger identisch ist, in der ein Reaktionssignal, vorzugsweise die optische Transmission, gemessen wird.

Als Reagenzien kommen dabei einerseits solche in Frage, die vom Trägermaterial ganz oder teilweise abgelöst werden können, z.B. Puffersubstanzen, Salze, Enzyme oder deren Substrate, andererseits solche, die am Trägermaterial adsorbtiv oder kovalent gebunden sind und an denen dann eine « Festphasen-Reaktion » stattfinden kann, beispielsweise Ionenaustauscher, trägergebundene biologisch aktive Substanzen wie Enzyme, Antikörper oder Antigene und ähnliche.

Für die Messung eines Reaktionssignals eignen sich z.B. ausser der schon erwähnten optischen Transmission je nach Ausführungsform der Messstelle auch Elektrodenpotentiale, elektrische Leitfähigkeit, Fluoreszenzstrahlung usw.

Im folgenden wird die Erfindung unter Bezugnahme auf die Zeichnung näher beschrieben. In dieser stellen dar:

Fig. 1 a und 1b Oben- bzw. Seitenansicht eines für die Erfindung geeigneten Einsatzelementes,

Fig. 2 Darstellung des Elementes von Fig. 1 a und 1b auf dem Rotor,

Fig. 3, 4 und 5 Ansichten eines anderen Analysenelementes zur Durchführung der Erfindung,

Fig. 6, 7, 8, 9 und 10 erfindungsgemäss erhaltene Analysenresultate in graphischer Darstellung.

Je nach den überlagerten Kräften ($K_2$) gibt es im wesentlichen zwei Ausführungsformen der Erfindung, wobei die treibenden Kräfte ($K_1$) jeweils Grenzflächen- bzw. Kapillarkräfte sind.

Bei der ersten Ausführungsform ist $K_2$ eine Zentrifugalkraft.

Bei diesem Analysensystem werden austauschbare Einsatzelemente für Zentrifugalanalysenrotoren, die beispielsweise aus einem Plastikformkörper aus Polystyrol, Plexiglas, Polyurethan u. ä. sowie Reagenzträgerfeldern, die aus einem saugfähigen Trägermaterial, das mit dem Reagenz imprägniert ist, oder anderen kleinen reagenzgefüllten Hohlräumen (z. B. einer Oberflächenstruktur im Plastikkörper), die in den Plastikkörper eingelegt sind, und einer Verschliessfolie bestehen, so auf einen Rotor einer Zentrifuge gesteckt, dass die flüssigkeitsbewegende Kapillarkraft von der Zentrifugalkraft gesteuert werden kann. Hierzu ist notwendig, dass verschiedene Drehzahlen und damit Zentrifugalkräfte eingestellt werden können.

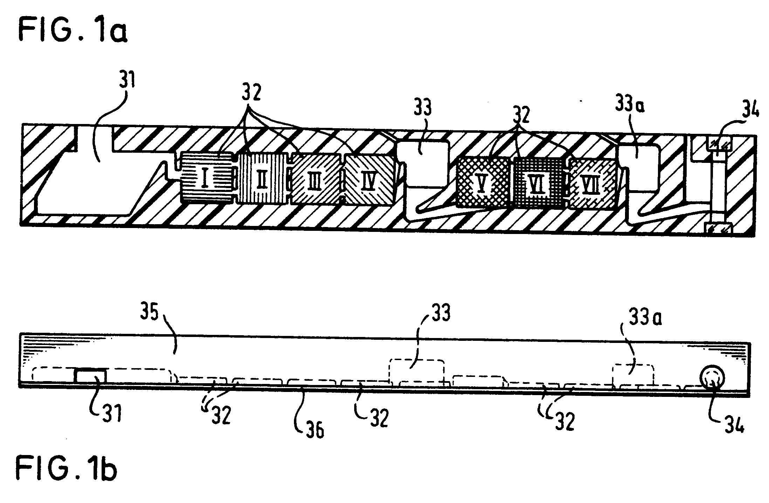

Der Analysenablauf bei dieser Ausführungsform der Erfindung wird anhand der Figuren 1a und 1b der beigefügten Zeichnung näher beschrieben. Fig. 1a zeigt ein für die Erfindung geeignetes Einsatzelement in der Aufsicht, Fig. 1b in der Seitenansicht im Schnitt.

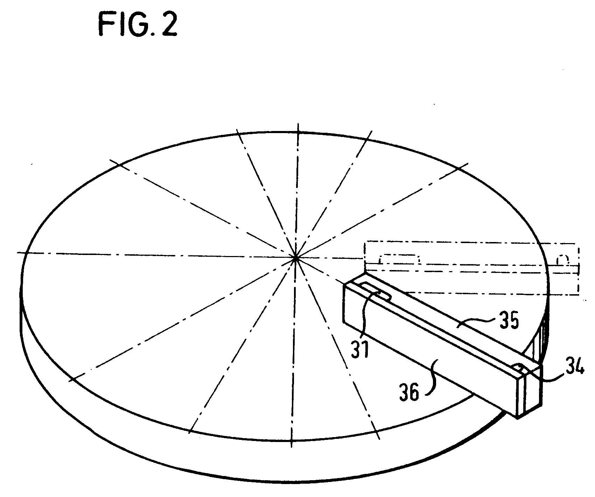

Fig. 2 stellt schematisch dar, wie das Einsatzelement von Fig. 1 auf einem geeigneten Zentrifugenrotor, wie er z.B. in der DE-OS 3 044 372 beschrieben ist, durch hier nicht gezeigte Befestigungsmittel aufgebracht ist.

Wie in Fig. 1 a gezeigt, ist in einem Plastikformkörper eine Probenauftragskammer 31 vorgesehen, die mit verschiedenen Reagenzfeldern I bis VII 32 in Verbindung steht. Jedes Reagenzfeld besteht aus einem mit einem bestimmten Reagenz imprägnierten Stück saugfähigen Träger, wie z.B. Papier oder Vlies. 33 und 33a sind Mischventile I und II, 34 bezeichnet die Messstelle (Küvette). Fig. 1b zeigt das Einsatzelement von Fig. 1 a in Seitenansicht. 35 bezeichnet den Plastikgrundkörper, 36 die Verschliessfolie durch die Probenauftragskammer, Reagenzfeldermischventile und Messstelle abgedeckt sind.

Die Durchführung des Verfahrens der Erfindung wird nun unter Bezugnahme auf Fig. 1 und 2 näher beschrieben.

Die Probe wird in die Probenauftragskammer 31 gegeben. Dann wird eine bestimmte Drehzahl U1 eingestellt, die geeignet ist, die Probe an das Reagenzfeld I32 heranzuführen. Sobald der Kontakt hergestellt ist, saugt die Kapillarkraft die Lösung auf, d.h. die Lösung wird über das Reagenzfeld 32 transportiert. Ist die Zen-

EP 0 073 513 B2

trifugalkraft Z1 kleiner als die Kapillarkraft K1, bleibt die Lösung, sofern das Aufnahmevolumen des Feldes grösser ist als das Volumen der aufgegebenen Probe, in dem Feld 32. Mit der Bedingung Z1 < K1 ist also die Verweilzeit der Lösung in RI genau festzulegen. Vergrössert man nun die Zentrifugalkraft auf Z2, so dass Z2 > K1 gilt, verlässt die um das auf RI befindliche Reagenz angereicherte Lösung dieses Feld und tritt mit dem Reagenzfeld R II in Kontakt. Hier wiederholt sich der Vorgang. Die Lösung wird über R II verteilt, d.h. weitertransportiert. In Analogie gelten die oben beschriebenen Bedingungen.

Der Vorgang lässt sich beliebig oft wiederholen, wobei im hier skizzierten Fall Reagenzfelder I bis IV durchlaufen werden. Natürlich können die Einsatzelemente anders gestaltet sein und auch mehr oder weniger Reagenzfelder aufweisen. Die Kräfte $K_n$ und $Z_n$ sind praktisch frei wählbar, was vor allem für letztere durch die stufenlose Einstellung von Z technisch sehr einfach realisiert werden kann. Die Durchführung von Analysenverfahren gewinnt dadurch an Vorteilen, dass man die dazu benötigte Zeit so kurz wie möglich hält. Deshalb sollte auch die Verweilzeit der Lösung auf den Reagenzfeldern so kurz wie möglich sein. Es ist möglich, die Zentrifugalkraft Z so gross zu wählen, dass die Lösung von den Kapillarkräften nur gebremst wird, d.h. die Lösung kommt nicht zum Stehen, sondern wandert mit von der Kraft $Z_n - K_n$ hervorgerufener Geschwindigkeit durch die entsprechenden Reagenzfelder. Findet dieser Vorgang in Sekunden oder Sekundenbruchteilen statt, so ist es leicht möglich, dass sich an der Lösungsfront höhere Reagenzkonzentrationen einstellen, d.h. in Richtung Zentrifugalkraft baut sich über das Lösungsvolumen ein Konzentrationsanstieg auf.

Zur Einhaltung definierter Reaktionsbedingungen gehört es jedoch, einheitliche Konzentrationsverhältnisse - wenn nötig - einzustellen. Um derartige Inhomogenitäten zu beseitigen, wird erfindungsgemäss ein sogenanntes Mischventil 33 vorgesehen. Das Mischventil 33 weist eine in Richtung der Zentrifugalkraft geschlossene Begrenzungswand auf. Am Boden ist eine schräg nach unten entgegen der Strömungsrichtung von Probenauftragskammer zu Messstelle angeordnete grenzflächenaktive Kammer, z.B. Kapillare angeordnet, welche an ihrem unteren Ende umbiegt und zum Reagenzfeld V weiterführt. Solange die Zentrifugalkraft Z3 grösser ist als die Kapillarkrak in der Bodenkapillare K3, wird die Flüssigkeit an der Begrenzungswand festgehalten. Senkt man Z3 unter den Wert von K3, so saugt die Kapillare die Flüssigkeit selbständig aus dem Mischraum 33 in die zugehörige Kapillare ab, wobei vorher bestehende Gradienten beseitigt werden und die Kapillarkraft transportiert die Flüssigkeit zum Reagenzfeld V. Allgemein ausgedrückt wirkt daher hier die Kapillarkraft in der Kapillare des Mischventils 33 immer als Transportkraft, wenn Z < K ist.

Die Standzeit im Mischventil 33 ist wiederum frei wählbar durch die Einstellung der Bedingung $Z_M > K_M$. Die Bedingung für den Transport ist also genau umgekehrt zu den oben beschriebenen Bedingungen.

Die weiteren Schritte über die Reagenzfelder V bis VII und über das Mischventil II 33a brauchen nicht erneut beschrieben werden, sie ergeben sich aus Analogiebetrachtungen zu den vorigen Schritten. In der erläuterten Ausführungsform ist das Mischventil 33a hilfreich, um eine homogene Lösung in die Küvette 34 zu befördern. In bekannter Ausführungsformen von Zentrifugalanalyzern ist das Mischen ein aufwendiger Vorgang, der z.B. durch starkes Beschleunigen und Abbremsen der Zentrifuge oder das Durchströmen der Lösung mit Luft bewirkt wird. Dies wird erfindungsgemäss mit technisch einfachen Mitteln vermieden.

Bei der zweiten Ausführungsform der Erfindung ist $K_2$ eine mechanische Druckkraft.

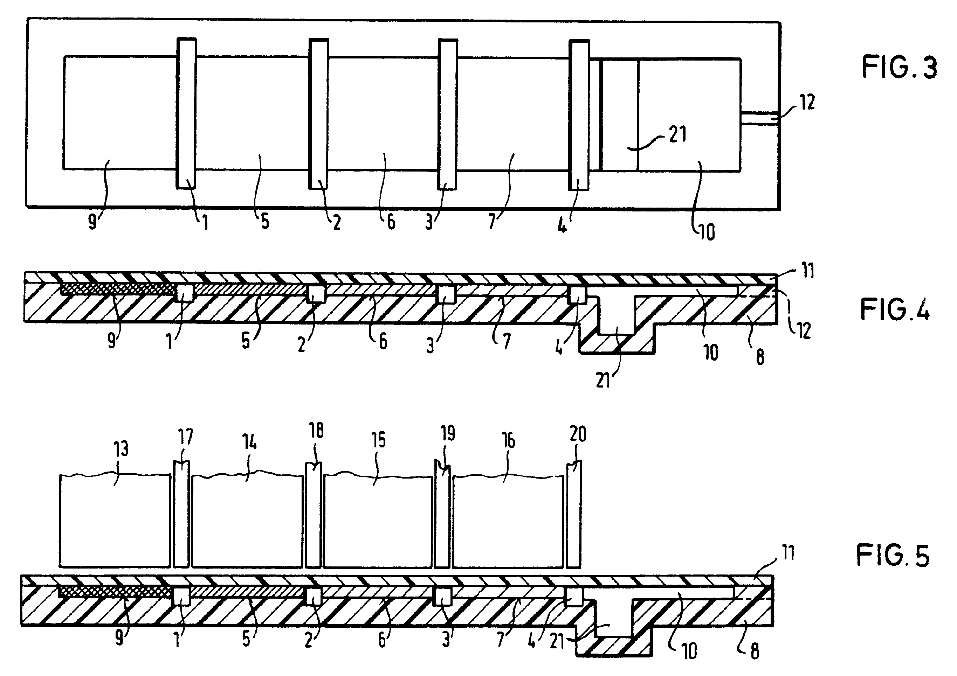

In den Fig. 3 und 4 der Zeichnung werden für diese Ausführungsform geeignete Mittel in Form von wegwerfbaren Analysenelementen dargestellt. In Fig. 5 wird eine das Anylysenelement enthaltende Vorrichtung zur Durchführung des erfindungsgemässen Verfahrens dargestellt.

In Fig. 3 ist ein derartiges Element in Aufsicht, in Fig. 4 in der Seitenansicht schematisch dargestellt. Der aus einem geeigneten Material, wie z.B. Plastik bestehende Formkörper 8 weist Reagenzträgerfelder 5, 6, 7 auf, die in den Plastikkörper eingelegt sind. Durch eine elastische Verschliessfolie 11 sind diese Reagenzträgei felder abgedeckt. Eine Probenauftragskammer 9 enthält ein saugfähiges inertes zusammenpressbares Material, das beispielsweise in entlastetem Zustand 15 $\mu$l Flüssigkeit aufnehmen kann und im zusammengepressten Zustand beispielsweise 2 $\mu$l Flüssigkeit zurückhält. Eine Ausbuchtungen im Körper 8 dient als Messstelle bzw. Küvette 21. Der Körper 8 weist ausserdem eine Überlaufkammer 10 und eine Entlüftungsbohrung 12 auf.

Ventilschlitze 1, 2, 3, 4 dienen zur Aufnahme von Ventilstempeln 17, 18, 19, 20, mit denen die einzelnen Reagenzträgerfelder voneinander getrennt werden können. Ausserdem sind Durckstempel 13, 14, 15, 16 vorgesehen, deren Grundfläche genau der Fläche der jeweiligen Reagenzträgerfläche entspricht und die dazu dienen, gefüllte Reagenzfelder zusammenzudrücken (Fig. 5).

Der Verfahrensablauf bei dieser Ausführungsform der Erfindung ist folgendermassen:

Die Probe wird in die Auftragskammer (9) gebracht, indem die Folie 11 mit einer Nadel durchstochen wird. Es wird eine solche Menge injiziert, dass sich das inerte, saugfähige Vlies in der Kammer 9 vollständig füllt, aber keine Lösung abgibt. Anschliessend wird z.B. durch eine geeignete Steuerungsvorrichtung, der Stempel 13 auf das Feld 9 gedrückt, so dass die Flüssigkeit dieses Feld verlassen muss und mittels der Kapillarkraft in das Feld 5 transportiert wird. Über die Kohäsion der Flüssigkeit wird diese praktisch vollständig nach 5 überführt. Anschliessend wird der Ventilstempel 17 z.B. ebenfalls über einen geeigneten Mechanismus gesteuert,

4

heruntergedrückt, so dass 5 von der Auftragskammer 9 abgetrennt ist. Die Folie 11 passt sich jeweils den Konturen an und dient als Dichtmembran. Die Probelösung kann eine frei wählbare Zeit lang im Feld 5 stehen gelassen werden. In der Regel löst sich allerdings das im Feld 5 befindliche Trockenreagenz innerhalb weniger Sekunden. Im nächsten Schritt wird der Stempel 14 auf das Feld 5 gedrückt, so dass die Flüssigkeit dieses Feld verlassen muss und über die kapillare Ansaugwirkung des Feldes 6 in dieses hineintransportiert wird. Danach wird der Ventilstempel 18 heruntergedrückt, so dass der Kontakt zu Feld 5 unterbrochen ist. Wiederum kann die Zeit zur Ab- oder Auflösung des Reagenz in Feld 6 frei gewählt werden (wobei es sich wiederum im allgemeinen um Sekunden dauernde Vorgänge handelt).

Sollte sich bei dem Flüssigkeitstransport durch Kapillarkräfte und bei dem Einströmen in das Reagenzfeld ein Konzentrationsgradient aufbauen, kann dieser dadurch beseitigt werden, dass man vor den Weitertransport nach Feld 7 einen Mischvorgang einschaltet. Durch Andrücken des Ventilstempels 19, Abheben des Ventilstempels 18 und des Stempels 14 und Andrücken des Stempels 15, wird die Flüssigkeit nach Feld 5 zurücktransportiert. Durch Anheben von Stempel 15 und Andrücken von Stempel 14 wird die Flüssigkeit wieder nach Feld 6 zurückbefördert. Gegebenenfalls kann diese Vor-Rück-Bewegung mehrere Male wiederholt werden. Zum Schluss wird dann wieder der Zustand hergestellt: Stempel 14 angedrückt, Ventilstempel 18 angedrückt.

Der Weitertransport der Flüssigkeit von Feld 6 nach Feld 7 erfolgt nach gegebenenfalls Abheben von Ventilstempel 19 in analoger Weise durch Andrücken von Ventilstempel 18 und Andrücken von Stempel 15. Gegebenenfalls kann der Mischvorgang zwischen Feld 6 und Feld 7 in Analogie zur beschriebenen Weise unter Benutzung von Ventilstempel 20 wiederholt werden.

Nach Auflösen des Reagenz in Feld 7 wird sie so entstandene Lösung dann durch Andrücken von Ventilstempel 19 und Andrücken von Stempel 15 in die Küvette 21 gedrückt.

Danach wird in geeigneter Weise in einem üblichen Verfahren die Extinktionsänderung während einer genügend langen Zeit gemessen. Aus dieser Signaländerungen kann ebenfalls mit üblichen Methoden die Konzentration der zu analysierenden Substanz berechnet werden.

Die für die Durchführung der für das Verfahren erforderliche Änderung der 1. bzw. 2. Kraft zur Verfügung stehenden Massnahmen sind für die Zentrifugalkraft in erster Linie in Drehzahländerungen, für die mechanische Druckkraft in Bewegung von Druckstempeln zu sehen. Die Grenzflächenkraft kann ausser durch die Oberflächengestaltung auch durch Einsatz oberflächenaktiver Mittel geregelt bzw. verändert werden. Als oberflächenaktive Mittel werden die Polyoxyäthylenderivate bevorzugt, jedoch können auch andere nichtionische Detergentien sowie anionische Detergentien, z. B. Gallensäurederivate, oder kationische Detergentien oder Gemische davon verwendet werden.

Beispiele für erfindungsgemäss durchführbare Analysen sind die in der DE-OS 3 044 385 beschriebenen. Insbesondere eignet sich das Verfahren zur Bestimmung von Glucose, Bilirubin, Creatinin, Albumin, Eiweiss, Eisen, Hemoglobin, Harnstoff, Harnsäure, Triglyceriden, Cholesterin, Chlorid, Kalzium, Phosphat, γ-GT, alkalischer Phosphate, GOT, GPT, Lactatdehydrogenase, Lipase, Amylase, Creatinkinase, Schilddrüsenhormonen, saure Phosphatase, Drogen, Krebsindikatoren und Gerinnungsfaktoren, wobei jeweils für diese Bestimmungen an sich bekannte Reagenzien eingesetzt werden können.

Die folgenden Beispiele erläutern die Erfindung weiter:

*Beispiel 1*

Glucosebestimmung unter Verwendung des Einsatzelementes gemäss Fig. 1 und 2.

Auf Filterpapiere der Grösse 6 × 6 mm und der Dicke 0,3 mm wurden folgende Reagenzien aufgebracht und wie aus Fig. 1 ersichtlich, im beschriebenen Einsatzelement positioniert:

| III | Natriumphosphatpuffer | 630 µg |
|---|---|---|
| | 2,4-Dichlorphenolsulfonsäure | 466 µg |
| | Tween 20 (Sorbinacrogollaurat) | 50 nl |
| | Mannit | 1 mg |
| IV | 4-Aminoantipyrin | 24 µg |
| VII | GOD (E.C. 1.1.3.4) | 2200 mU |
| | POD (E.C. 1.11.1.7) | 400 mU |

Humanserumproben wurden 1 : 200 mit bidest. Wasser verdünnt. Von dieser verdünnten Lösung wurden 60 µl in die Probenauftragskammer 1 gegeben.

Es wurde bei 25°C nach folendem Programm zentrifugiert:

| | | | |
|---|---|---|---|
| 1. | 10 sec | 180 Upm | Anfeuchten der ersten Vliese |
| 2. | 10 sec | 1500 Upm | Ausschleudern in 1. Mischventil 3 |
| 3. | 15 sec. | 0 Upm | Überführung nach V |
| 4. | 10 sec | 1500 Upm | Ausschleudern in 2. Mischventil 3a |
| 5. | 15 sec | 0 Upm | Entleeren des 2. Mischventils 3a |

| | | | |
|---|---|---|---|
| 6. | 10 sec | 150 Upm | Überführen in die Küvette 4 |
| 7. | 5 sec | 1500 Upm | Austreiben von Luftblasen |
| 8. | 225 sec | 360 Upm | Messung bei 500 nm |

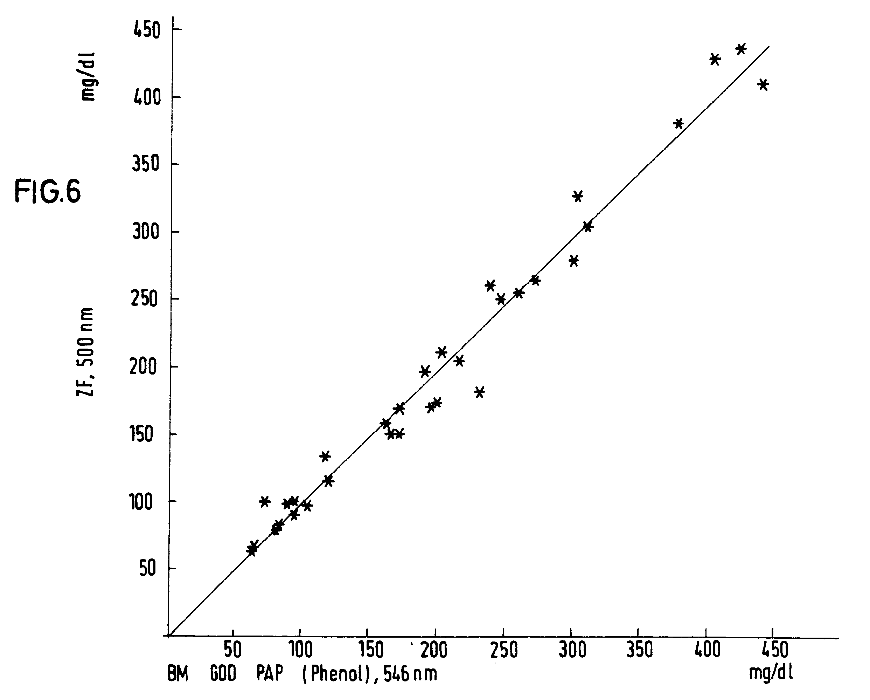

Die Änderung der Extinktion in Abhängigkeit von der Zeit wurde gemessen und nach einem der üblichen « Fixed-time-kinetischen Verfahren » ausgewertet. Die unbekannten Konzentrationen an Glucose in der Probe wurden nach Eichung des Verfahrens mit einem Standard bestimmt. Die Übereinstimmung mit einer Vergleichsmethode, der eine der bekannten manuellen Techniken zugrunde liegt, ist sehr gut, wie aus Fig. 6 zu erkennen ist, welche die Ergebnisse der Vergleichsmethode auf der Abscisse, der erfindungsgemässen Methode auf der Ordinate zeigt.

Auf der Ordinate sind auch in den folgenden Beispielen die Werte mit der erfindungsgemässen Methode aufgetragen (Symbol ZF).

*Beispiel 2*

Alkalische Phosphatase wurde auf dem Einsatzelement gemäss Fig. 1 und 2 bestimmt.

Auf Filterpapiere der Grösse 6 × 6 mm und der Dicke 0,3 mm wurden folgende Reagenzien aufgebracht und wie aus Fig. 1 ersichtlich, positioniert:

| | | |
|---|---|---|
| II | Natriumcarbonatpuffer | 1200 $\mu$g |
| | Magnesiumaspartat | 16 $\mu$g |
| III | Natriumcarbonatpuffer | 1200 $\mu$g |
| | Magnesiumaspartat | 16 $\mu$g |
| VI | Tris-p-Nitrophenylphosphat | 313 $\mu$g |
| | Tris | 31 $\mu$g |

Eine Humanserumprobe wurde 1 : 10 mit bidest. Wasser verdünnt. Von dieser verdünnten Lösung wurden 60 $\mu$l in die Probenauftragskammer 1 gegeben.

Es wurde bei 37°C nach folgendem Programm zentrifugiert:

| | | | |
|---|---|---|---|
| 1. | 10 sec | 180 Upm | Anfeuchten der ersten Vliese |
| 2. | 10 sec | 1500 Upm | Ausschleudern in 1. Mischventil 3 |
| 3. | 15 sec. | 0 Upm | Überführung nach V |
| 4. | 10 sec | 1500 Upm | Ausschleudern in 2. Mischventil 3a |
| 5. | 15 sec | 0 Upm | Entleeren des 2. Mischventils 3a |
| 6. | 10 sec | 150 Upm | Überführen in die Küvette 4 |
| 7. | 5 sec | 1500 Upm | Austreiben von Luftblasen |
| 8. | 225 sec | 360 Upm | Messung bei 410 nm |

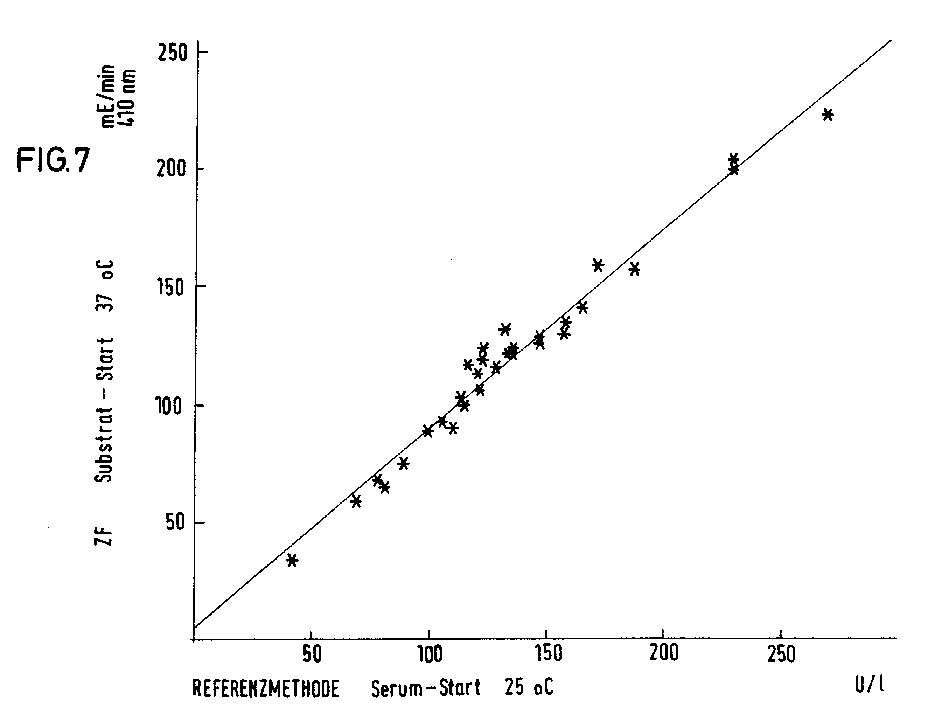

Die aufgezeichneten Abhängigkeiten der Extinktionen von der Zeit wurden nach einem der üblichen Verfahren, bei denen die Steigung der Geraden ein Mass für die Aktivität des zu bestimmenden Enzyms ist, ausgewertet. Die unbekannten Aktivitäten an alkalischer Phosphatase in der Probe wurden nach Eichung des Verfahrens mit einem Standard bestimmt. Die Korrelation mit einer Vergleichsmethode, der eine der bekannten manuellen Techniken zugrunde liegt, ist gut, wie aus Fig. 7 zu erkennen ist.

*Beispiel 3*

Bilirubin-Bestimmung mit dem Einsatzelement von Fig. 1 und 2.

Auf Filterpapiere der Grösse 6 × 6 mm und der Dicke 0,3 mm wurden folgende Reagenzien aufgebracht und wie aus Fig. 1 ersichtlich, positioniert:

| | | |
|---|---|---|
| II | 2,5-Dichlorphenyldiazonium-naphtolsulfonat | 68 $\mu$g |
| III | Cetylpyridiniumchlorid | 1600 $\mu$g |
| | Weinsäure | 2400 $\mu$g |
| V | Nicht imprägniertes Filterpapier | |

Serumproben wurden 1 : 10 mit bidest. Wasser verdünnt. Von dieser verdünnten Lösung wurden je 60 $\mu$l in die Probenauftragskammer 1 gegeben.

Es wurde bei 25°C nach folgendem Programm zentrifugiert:

| | | | |
|---|---|---|---|
| 1. | 10 sec | 180 Upm | Anfeuchten der ersten Vliese |
| 2. | 10 sec | 1500 Upm | Ausschleudern in 1. Mischventil 3 |
| 3. | 15 sec. | 0 Upm | Überführung nach V |
| 4. | 10 sec | 1500 Upm | Ausschleudern in 2. Mischventil 3a |
| 5. | 15 sec | 0 Upm | Entleeren des 2. Mischventils 3a |
| 6. | 10 sec | 150 Upm | Überführen in die Küvette 4 |
| 7. | 5 sec | 1500 Upm | Austreiben von Luftblasen |
| 8. | 225 sec | 360 Upm | Messung bei 550 nm |

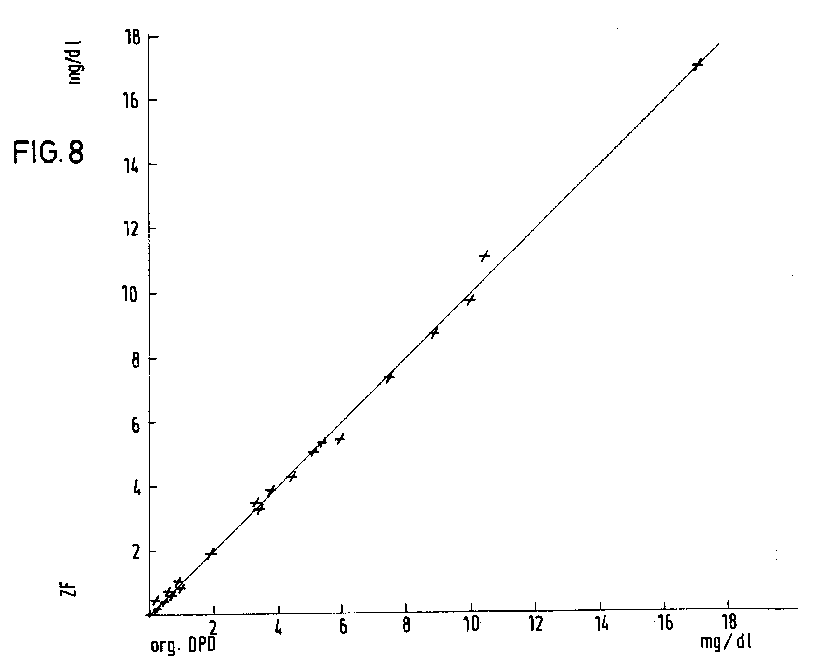

Die aufgezeichneten Abhängigkeiten der Extinktionen von der Zeit wurden nach einem der üblichen « Endpunkt-Verfahren » ausgewertet; die unbekannten Konzentrationen an Bildrubin in der Probe wurden nach Eichung des Verfahrens mit einem Standard bestimmt. Die Übereinstimmung mit einer Vergleichsmethode, der eine der bekannten manuellen Techniken zugrunde liegt, ist sehr gut, wie Fig. 8 zeigt.

*Beispiel 4*

Creatinkinase-Bestimmung mit dem Einsatzelement von Fig. 1 und 2.

Auf Filterpapiere der Grösse 6 × 6 mm und der Dicke 0,3 mm wurden folgende Reagenzien aufgebracht und wie aus Fig. 1 ersichtlich, positioniert:

| IV | Imidazol | 424 $\mu$g |
|---|---|---|
| | Glucose | 240 $\mu$g |
| | Magnesiumchlorid · 6H$_2$O | 128 $\mu$g |
| | EDTA-Natrium-Salz | 47 $\mu$g |
| | N-Acetylcystein | 205 $\mu$g |
| | Adenosinmonophosphat-Natrium-Salz | 157 $\mu$g |
| | Adenosindiphosphat | 54 $\mu$g |
| | Diadenosinpentaphosphat-Lithium-Salz | 0,6 $\mu$g |
| | NADP-Natrium-Salz | 110 $\mu$g |

| V | Hexokinase (E.C. 2.7.1.1.) | 218 mU |
|---|---|---|
| | Glucose-6-Phosphat-Dehydrogenase (E.C. 1.1.1.49) | 123 mU |
| | Creatinphosphat-Natrium-Salz | 615 $\mu$g |

Eine Humanserumprobe wurde 1 : 25 mit bidest. Wasser verdünnt. Von dieser verdünnten Lösung wurden 60 μl in die Probenauftragskammer 1 gegeben.

Es wurde bei 37°C nach folgendem Programm zentrifugiert:

| 1. | 10 sec | 180 Upm | Anfeuchten der ersten Vliese |
|---|---|---|---|
| 2. | 10 sec | 1500 Upm | Ausschleudern in 1. Mischventil 3 |
| 3. | 15 sec. | 0 Upm | Überführung nach V |
| 4. | 10 sec | 1500 Upm | Ausschleudern in 2. Mischventil 3a |
| 5. | 15 sec | 0 Upm | Entleeren des 2. Mischventils 3a |
| 6. | 10 sec | 150 Upm | Überführen in die Küvette 4 |
| 7. | 5 sec | 1500 Upm | Austreiben von Luftblasen |
| 8. | 225 sec | 360 Upm | Messung bei 340 nm |

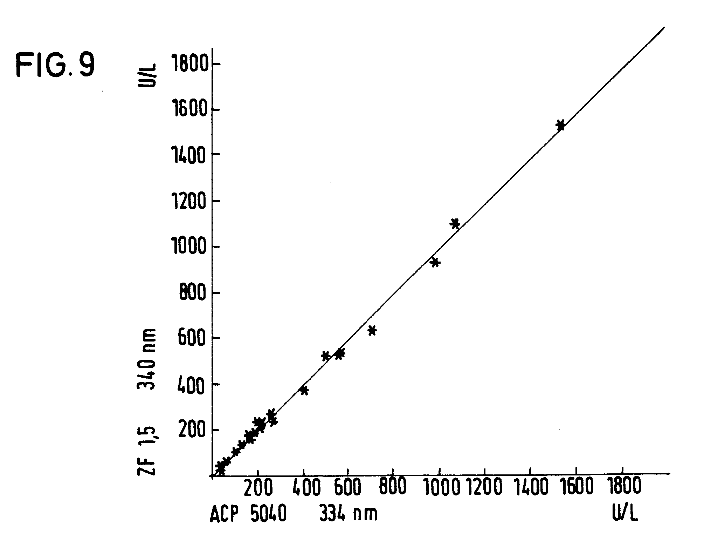

Die aufgezeichneten Abhängigkeiten der Extinktionen von der Zeit wurden nach einem der üblichen Verfahren für kinetische Messungen ausgewertet, die unbekannten Aktivitäten an Creatinkinase in der Probe wurden nach Eichung des Verfahrens mit einem Standard bestimmt. Die Übereinstimmung mit einer Vergleichsmethode, der eine der bekannten manuellen Techniken zugrunde liegt, ist sehr gut, wie Fig. 9 zeigt.

*Beispiel 5*

IgG-Bestimmung mit dem Einsatzelement von Fig. 1 und 2.

Auf Filterpapiere der Grösse 6 × 6 mm und der Dicke 0,3 mm wurden folgende Reagenzien aufgebracht und wie aus Fig. 1 eisichtlich, positioniert (in diesem Fall wurden zwei verschiedene Reagenzträgerpapiere in die gleiche Kammer gegeben):

| V | Natriumhydrogen- | |
|---|---|---|
| | phosphat · $2H_2O$ | 620 µg |
| | Kaliumhydrogenphosphat | 107 µg |
| | Polyäthylenglycol 6000 | 1570 µg |
| V | Antikörper gegen IgG | |
| | (Titer = 16 mg/ml) | 258 µg |

Eine Humanserumprobe wurde 1 : 200 mit bidest. Wasser verdünnt. Von dieser verdünnten Lösung wurden 60 µl in die Probenauftragskammer 1 gegeben.

Es wurde bei 25°C nach folgendem Programm zentrifugiert:

1. 10 sec 180 Upm Anfeuchten der ersten Vliese
2. 10 sec 1500 Upm Ausschleudern in 1. Mischventil 3
3. 15 sec. 0 Upm Überführung nach V

4. 10 sec 1500 Upm Ausschleudern in 2. Mischventil 3a
5. 15 sec 0 Upm Entleeren des 2. Mischventils 3a
6. 10 sec 150 Upm Überführen in die Küvette 4
7. 5 sec 1500 Upm Austreiben von Luftblasen
8. 225 sec 360 Upm Messung bei 340 nm

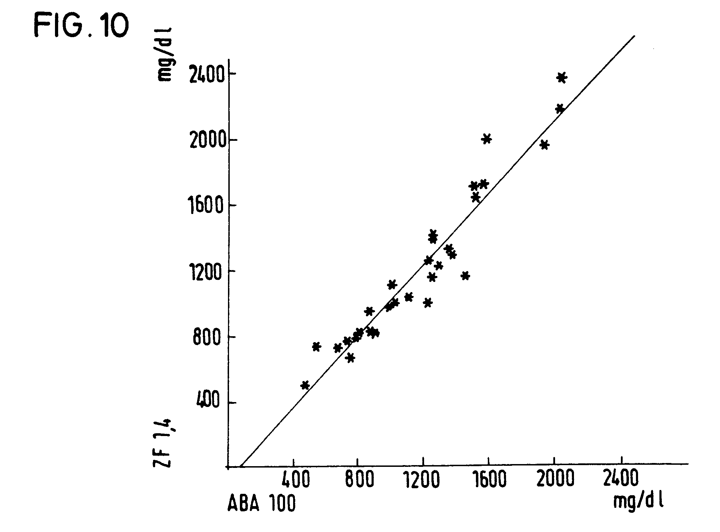

Die aufgezeichneten Abhängigkeiten der Extinktionen von der Zeit wurden nach einem der üblichen Verfahren zur Auswertung von kinetischen Trübungstesten ausgewertet, die unbekannten Konzentrationen an IgG in der Probe wurden nach Eichung des Verfahrens mit 3 Standards unterschiedlicher Konzentration und Erstellung einer Eichkurve bestimmt. Die Übereinstimmung mit einer Vergleichsmethode, der eine Adaptation auf dem Analysenautomaten « ABA 100 » der Firma ABBOTT zugrunde liegt, ist sehr gut, wie Fig. 10 zeigt.

*Beispiel 6*

Glucosebestimmung mit dem Analysenelement gemäss Fig. 3.

Reagenzträgerpapiere der Grösse 6 × 6 × 0,3 mm werden wie folgt hergestellt: 10 µl der Reagenzlösungen, die ein Viertel der Substanzmengen enthalten wie die verschiedenen Papiere des Beispiels in Beispiel 1, werden auf ein Papier der angegebenen Grösse gegeben. Die Lösungen werden vollständig aufgesaugt; anschliessend wird das Lösungsmittel Wasser durch Lyophilisieren aus den Papieren entfernt.

Die drei verschiedenen Papiere werden in der gleichen Reihenfolge wie in Beispiel 1 auf die drei Positionen des Analysenelements von Fig. 3 gelegt.

Die Probe wird 1 : 200 mit bidest. Wasser verdünnt. 15 µl dieser verdünnen Lösung werden in die Auftragskammer 9 gegeben. Dann wird die Lösung in der oben geschilderten Weise auf den ersten Reagenzträger 5 gebracht. Verweilzeit: 10 sec. Anschliessend wird die Lösung aus 5 nach 6 in ebenfalls bereits geschilderter Art und Weise gebracht. Verweilzeit: 10 sec. In analoger Weise wird die Lösung auf den Reagenzträger 3 (7) gebracht. Verweilzeit: 5 sec. Von dort wird die Lösung durch langsames Absenken des Stempels 16 in die Küvette 21 gebracht. In ihr wird in bekannter Weise die Extinktion bei 500 nm in Abhängigkeit von der Zeit verfolgt. Aus dem Verlauf dieser Kurve kann mit dem bekannten « Fixed-time-kinetischen » Verfahren nach Eichung mit einem Standard die Glukosekonzentration in unbekannten Proben bestimmt werden.

Es wurden wässrige Lösungen mit den Konzentrationen 50, 100, 150, 200, 300 und 400 mg/dl (durch Einwaage eingestellt) untersucht. Die Wiederfindung lag zwischen 98 und 102%.

*Beispiel 7*

Alkalische Phosphatase mit dem Element gemäss Fig. 3.

Reagenzträgerpapiere der Grösse 6 × 6 × 0,3 mm werden wie folgt hergestellt: 10 µl der Reagenzlösungen,

9

die genau ein Viertel der Substanzmengen enthalten wie die verschiedenen Papiere des Beispiels 2, werden auf ein Papier der angegebenen Grösse gegeben. Die Lösungen werden vollständig aufgesaugt; anschliessend wird das Lösungsmittel Wasser durch Lyophilisieren aus den Papieren entfernt.

Die beiden Papiere werden in der gleichen Reihenfolge wie in Beispiel 2 auf die ersten beiden Positionen des Analysenelements von Fig. 3 gelegt. Auf die dritte Position wird ein Papier gelegt, das kein Reagenz enthält.

Die Probe wird 1 : 10 mit bidest. Wasser verdünnt. 15 µl dieser verdünnten Lösung werden in die Auftragskammer 9 gegeben. Dann wird die Lösung in der geschilderten Weise auf den ersten Reagenzträger 5 gebracht. Verweilzeit: 10 sec. Anschliessend wird die Lösung aus 5 nach 6 in ebenfalls bereits geschilderter Art und Weise gebracht. Verweilzeit: 10 sec. In analoger Weise wird die Lösung auf das Leervlies 7 gebracht. Verweilzeit: 5 sec. Von dort wird die Lösung durch langsames Absenken des Stempels 16 in die Küvette 21 gebracht. In ihr wird die Extinktion bei 410 nm in Abhängigkeit von der Zeit verfolgt. Aus dem Verlauf der aufgezeichneten Geraden kann mit bekannten kinetischen Bestimmungsverfahren nach Eichung mit einen Standard die Aktivität der alkalischen Phosphatase in unbekannten Proben bestimmt werden.

Es wurden verschiedene Kontrollseren, die Aktivitäten zwischen 30 und 600 U/l enthielten, untersucht. Die Wiederfindung lag zwischen 90 und 110%.

*Beispiel 8*

Creatinkinasebestimmung mit dem Element gemäss Fig. 3.

Reagenzträgerpapiere der Grösse $6 \times 6 \times 0,3$ mm werden wie folgt hergestellt: 10 µl der Reagenzlösungen, die genau ein Viertel der Substanzmengen enthalten wie die verschiedenen Papiere des Beispiels 4, wurden auf ein Papier der angegebenen Grösse gegeben. Die Lösungen werden vollständig aufgesaugt; anschliessend wurde das Lösungsmittel Wasser durch Lyophilisieren aus den Papieren entfernt.

Die beiden Papiere wurden in der gleichen Reihenfolge wie in Beispiel 4 auf die ersten beiden Positionen des Analysenelements von Fig. 3 gelegt. Auf die dritte Position wurde ein Papier der gleichen Art, das kein Reagenz enthielt, gelegt.

Die Probe wurde 1 : 25 mit bidest. Wasser verdünnt. 15 µl dieser verdünnten Lösung wurden in die Auftragskammer 9 gegeben. Dann wird die Lösung in der geschilderten Weise auf den ersten Reagenzträger 5 gebracht. Verweilzeit: 10 sec. Anschliessend wurde die Lösung aus 5 nach 6 in ebenfalls bereits geschilderter Art und Weise gebracht. Verweilzeit: 10 sec. In analoger Weise wurde die Lösung auf das Feld 7 bewegt. Verweilzeit: 5 sec. Von dort wird die Lösung durch langsames Absenken des Stempels 16 in die Küvette 21 gebracht. In ihr wird in bekannter Weise die Extinktion bei 340 nm in Abhängigkeit von der Zeit verfolgt. Nach einer gekrümmten Anfangsphase erhielt man einen linearen Verlauf dieser Funktion. Aus diesem Anteil wird mit Hilfe von bekannten Auswerteverfahren für kinetische Methoden nach Eichung mit einem Standard die Aktivität an Creatinkinase in einer unbekannten Probe bestimmt.

Es wurden verschiedene Humanserumproben mit aufgereinigtem Enzym in der Weise aufgestockt, dass man Aktivitäten von 5 bis 800 U/l erhielt. Vergleichswerte wurden durch eine manuelle Messung gewonnen. Die Wiederfindungen gegenüber diesen manuellen Werten lagen zwischen 92 und 110%.

## Patentansprüche

1. Verfahren zur Durchführung analytischer Bestimmungen durch Mischen und Inkubieren einer Probelösung mit wenigstens einem Reagenz und Messung eines Parameters im Reaktionsgemisch, wobei die Probelösung von einer Aufgabestelle zu einer Messstelle transportiert wird, dadurch gekennzeichnet, dass man die Probelösung zuerst zu einem löslichen Trockenreagenz transportiert unter wenigstens teilweise Auflösung des letzteren und dann zur Messstelle weitertransportiert und der Transport durch zwei verschiedene Kräfte erfolgt, wobei er wenigstens auf einem Teil der Transportstrecke duch eine auf die Lösung wirkende Grenzflächenkraft als erste Kraft bewirkt wird, der zur Regelung der Transportgeschwindigkeit oder Transportrichtung als zweite Kraft eine Zentrifugalkraft oder mechanische Druckkraft überlagert wird, die je nachdem, welcher Transportzustand der Flüssigkeit eingestellt werden soll, grösser oder kleiner als die erste Kraft gemacht wird.

2. Verfahren nach Anspruch 1, dadurch gekennzeichnet, dass man die erste physikalische Kraft durch die Oberflächengestaltung oder/und oberflächenaktive Mittel regelt.

3. Verfahren nach einem der Ansprüche 1 bis 2, dadurch gekennzeichnet, dass man den Wert der zweiten physikalischen Kraft mehrmals höher oder niedriger als den Wert der ersten physikalischen Kraft einstellt.

4. Verfahren nach einem der vorhergehenden Ansprüche, dadurch gekennzeichnet, dass man während der Inkubation des Reagenz-Probelösungsgemisches den Wert der zweiten physikalischen Kraft über den Wert der ersten physikalischen Kraft einstellt.

5. Verfahren nach einem der Ansprüche 2 bis 4, dadurch gekennzeichnet, dass man die zweite physikalische Kraft durch Änderung der Drehzahl eines Zentrifugenrotors erhöht oder erniedrigt.

6. Rotoreinsatzelement zur Durchführung des Verfahrens nach den Ansprüchen 1 bis 5 mit einer Zentrifugalkraft als zweiter Kraft, gekennzeichnet durch einen Formkörper (35) mit einer Probenauftragskammer (31), die mit einer Mehrzahl von Reagenzfeldern (32) in Verbindung steht, die jeweils ein mit einem bestimmten Reagenz imprägniertes saugfähiges Trägermaterial enthalten, wenigstens einer Mischventilkammer (33, 33a), einer Messkammer (34) und Mitteln (36) zum Verschliessen der Kammern und Felder.

7. Vorrichtung zur Duchführung des Verfahrens oder nach den Ansprüchen 1 bis 4 mit einer mechanischen Druckkraft als zweiter Kraft, gekennzeichnet durch einen Formkörper (8) mit einer Probenauftragskammer (9), in der sich ein saugfähiges, inertes zusammenpressbares Material befindet, einer Mehrzahl von Reagenzträgerfeldern (5, 6, 7), die jeweils ein mit einem bestimmten Reagenz imprägniertes saugfähiges zusammenpressbares Trägermaterial enthalten, einer Messkammer (21), einer Überlaufkammer (10) und einer Entlüfungsbohrung (12), sowie mit zwischen den einzelnen Reagenzträgerfeldern (5, 6, 7) sowie der Auftragskammer (9) angeordneten Ventilschlitzen (1, 2, 3, 4) mit darin beweglich angeordneten Ventilstempeln (17, 18, 19, 20) sowie weiter einer Mehrzahl von Druckstempeln (13, 14, 15, 16), die so angeordnet sind, dass sie unabhängig voneinander auf die Probenauftragskammer (9) und die Reagenzträgerfelder (5, 6, 7) eine bestimmte Druckkraft auszuüben vermögen.

## Claims

1. Process for the carrying out of analytical determinations by mixing and incubating a sample solution with at least one reagent and measuring a parameter in the reaction mixture, whereby the sample solution is transported from an application point to a measurement point, characterised in that one first transports a sample solution to a soluble dry reagent, with at least partial dissolving of the latter, and then further transports to the measurement point and the transport takes place by the different forces, whereby at least on a part of the transport path, it is brought abut by a boundary surface force acting on the solution as a first force which, for the regulation of the transport velocity or transport direction, is superimposed by a centrifugal force or mechanical pressure force as a second force which, depending upon which transport state of the fluid is to be regulated, is made greater or smaller than the first force.

2. Process according to claim 1, characterised in that one regulates the first physical force by the surface construction and/or surface-active agents.

3. Process according to claim 1 or 2, characterised in that one regulates the value of the second physical force several times higher or lower than the value of the first physical force.

4. Process according to one of the preceding claims, characterised in that, during the incubation of the reagent-sample solution mixture, one regulates the value of the second physical force above the value of the first physical force.

5. Process according to one of claims 2 to 4, characterised in that one increases or lowers the second physical force by alteration of the speed of rotation of a centrifugal rotor.

6. Rotor insert element for the carrying out of the process according to claims 1 to 6 with a centrifugal force as second force, characterised by a formed body (35) with a sample application chamber (31), which is in connection with a plurality of reagent zones (32), each of which contains an absorbent material impregnated with a particular reagent, at least one mixing valve chamber (33, 33a), a measurement chamber (34) and means (36) for the closure of the chambers and zones.

7. Device for the carrying out of the process according to claims 1 to 4 with a mechanical pressure force as the second force, characterised by a formed body (8) with a sample application chamber (9) in which is present an absorbent, inert compressable material, a plurality of reagent carrier xones (5, 6, 7), each of which contains an absorbent compressable carrier material impregnated with a particular reagent, a measurement chamber (21), an overflow chamber (10) and a ventilation bore (12), as well as valve slots (1, 2, 3, 4) arranged between the individual reagent carrier zones (5, 6, 7), as well as the application chamber (9), with movable valve plungers (17, 18, 19, 20) arranged therein, as well as a plurality of pressure plungers (13, 14, 15, 16) which are so arranged that, independently of one another, they are able to exert a particular pressure force on the sample application chamber (9) and the reagent carrier zones (5, 6, 7).

## Revendications

1. Procédé d'exécution de déterminations analytiques par mélange et incubation d'une solution d'échantillon avec au moins un réactif et mesure d'un paramètre dans le mélange réactionnel, la solution d'échantillon étant transportée d'un emplacement de chargement à un emplacement de mesure, caractérisé en ce qu'on transporte d'abord la solution d'échantillon vers un réactif sec soluble avec dissolution au moins partielle de ce dernier, puis on la transporte plus loin jusqu'à l'emplacement de mesure et le transport s'effectue grâce à deux forces différentes, étant provoqué au-moins sur une partie de la distance de transport par une force de surface agissant sur la solution en tant que première force, à laquelle, pour le réglage de la vitesse de transport ou de la direction de transport, est superposée comme deuxième force une force centrifuge et/ou une force de pression mécanique laquelle, selon l'état de transport du liquide qui doit être réglé, est rendue plus élevée ou plus faible que la première force.

2. Procédé suivant la revendication 1, caractérisé en ce qu'on règle la première force physique par la configuration superficielle et/ou un agent tensio-actif.

3. Procédé suivant l'une des revendications 1 à 2, caractérisé en ce qu'on règle la valeur de la deuxième force physique à une valeur plusieurs fois supérieure ou inférieure à la valeur de la première force physique.

4. Procédé suivant l'une des revendications précédentes, caractérisé en ce que, pendant l'incubation du mélange réactif-solution d'échantillon, on règle la valeur de la deuxième force physique au-dessus de la valeur de la première force physique.

5. Procédé suivant l'une des revendications 2 à 4, caractérisé en ce qu'on élève ou abaisse la deuxième force physique par modification du nombre de tours d'un rotor de centrifugeuse.

6. Elément d'insertion dans un rotor pour l'exécution du procédé selon les revendications 1 à 5 avec une force centrifuge comme deuxième force, caractérisé par une pièce moulée (35) avec une chambre de chargement des échantillons (31), qui est en communication avec une multiplicité de champs de réactifs (32) qui contiennent, à chaque fois, une matière de support absorbante imprégnée d'un réactif déterminé, par au moins une chambre de soupape de mélange (33, 33a), par une chambre de mesure (34) et par un moyen (36) pour l'obturation des chambres et des champs.

7. Dispositif pour l'exécution du procédé suivant les revendications 1 à 4 avec une force de pression mécanique comme seconde force, caractérisé par une pièce moulée (8) avec une chambre de chargement des échantillons (9),dans laquelle se trouve une matière inerte, comprimable, absorbante,une multiplicité de champs de support de réactif (5, 6, 7), qui contiennent à chaque fois une matière de support absorbante, comprimable, imprégnée d'un réactif déterminé, une chambre de mesure (21), une chambre à trop-plein (10) et un percement d'évacuation de l'air (12), ainsi qu'avec des fentes de soupape (1,2,3,4) disposées entre les divers champs de supports de réactifs (5,6,7) ainsi que la chambre de chargement (9) avec des poinçons de soupape (17, 18, 19, 20) qui y sont disposés de façon mobile ainsi qu'en outre une multiplicité de poinçons de pression (13, 14, 15, 16) qui sont disposés de telle sorte qu'ils peuvent exercer indépendamment les uns des autres, sur la chambre de chargement des échantillons (9) et sur les champs des supports des réactifs (5, 6, 7), une force de pression déterminée.

FIG. 1a

FIG. 1b

FIG. 2

FIG.3

FIG.4

FIG.5

FIG.6

FIG.7

EP 0 073 513 B2

FIG.8

FIG.9

FIG. 10

EP 0 073 513 B2